# EUROPÄISCHE PATENTANMELDUNG

(11) **EP 0 810 202 A2**
(43) Veröffentlichungstag der Anmeldung: **03.12.1997**
(21) Anmeldenummer: 97108445.4
(22) Anmeldetag: 26.05.1997
(51) Int. Cl.: C07C 217/10

(54) **Verfahren zur Herstellung von 4-Oxa-Aminen**

(30) Priorität: 30.05.1996 DE 19621704
(71) Anmelder: HOECHST AKTIENGESELLSCHAFT, 65926 Frankfurt am Main (DE)
(72) Erfinder: Hölderich, Wolfgang, Prof. Dr., 67227 Frankenthal (DE); Paczkowski, Marcus, Dr., 64289 Darmstadt (DE); Heinz, Dieter, 40670 Meerbusch (DE)

(57) **Zusammenfassung**

Gegenstand der Erfindung ist ein Verfahren zur Herstellung von 4-Oxa-Aminen. Es besteht in der Umsetzung von 1,3-Dioxanen mit Ammoniak und Wasserstoff in Gegenwart von Hydrierkatalysatoren.

## Beschreibung

Die vorliegende Erfindung betrifft ein neues Verfahren zur Herstellung von 4-Oxa-Aminen. Ausgangsverbindungen sind leicht zugängliche 1,3-Dioxane, die in Gegenwart von Hydrierkatalysatoren mit Ammoniak und Wasserstoff umgesetzt werden (reduktive Aminierung).

4-Oxa-Amine sind technisch wertvolle Zwischenprodukte für die Synthese von Agrochemikalien, Pharmazeutika, Lösungsmitteln und Rohstoffen für die Waschmittelindustrie. Die Herstellung der Amine kann aus den entsprechenden 4-Oxa-Alkoholen durch nukleophile Substitution erfolgen oder durch reduktive Aminierung von 4-Oxa-Aldehyden. Bei beiden Verfahren handelt es sich um zweistufige Prozesse, in deren Verlauf zunächst der Alkohol bzw. der Aldehyd hergestellt werden müssen. In einer zweiten Reaktionsstufe erfolgt dann die Umwandlung von Alkohol oder Aldehyd in die Aminoverbindungen. Eine derartige Arbeitsweise ist technisch aufwendig und genügt daher nicht immer den wirtschaftlichen Anforderungen an technische Prozesse.

Es bestand daher die Aufgabe ein Verfahren bereitzustellen, das die aufgezeigten Nachteile vermeidet, technisch einfach zu realisieren und allgemein anwendbar ist. Es soll überdies selektiv arbeiten und hohe Ausbeuten der Zielprodukte sicherstellen.

Die Erfindung besteht in einem Verfahren zur Herstellung von 4-Oxa-Aminen der allgemeinen Formel (I)

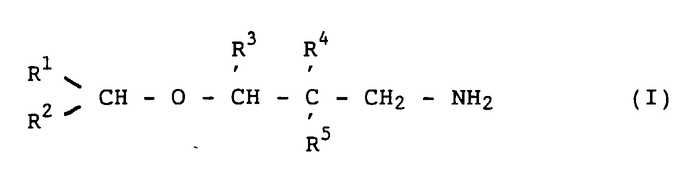

Es ist dadurch gekennzeichnet, daß man 1,3-Dioxane der allgemeinen Formel (II) 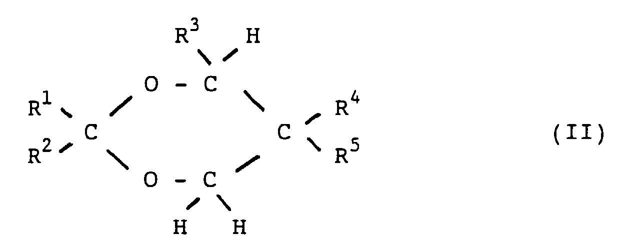 in der R¹, R², R⁴ und R⁵ gleich oder verschieden sind und Wasserstoff, geradkettige oder verzweigte Alkyl-, Alkenyl- oder Alkinylreste mit bis zu 18 Kohlenstoffatomen, Cycloalkyl- oder Cycloalkenylreste mit 5 bis 8 Kohlenstoffatomen, Aryl- Alkylaryl-, Aralkyl-, Aralkenyl- oder Alkenylarylreste mit 6 bis 16 Kohlenstoffatomen oder heterocyclische Reste bedeuten, die Reste R¹ und R² und/oder R⁴ und R⁵ zusammen mit dem Kohlenstoffatom, an das sie gebunden sind, ein Cycloalkan, Cycloalken oder einen Heterocyclus mit 5 bis 7 Ringgliedern bilden können, wobei R¹, R², R⁴ und R⁵ überdies Substituenten, insbesondere solche, die unter den Reaktionsbedingungen inert sind, tragen können und R³ für Wasserstoff oder einen geradkettigen oder verzweigten Alkylrest steht bei Drücken von 0,1 bis 35 MPa und Temperaturen von 40 bis 500°C in Gegenwart eines Hydrierkatalysators mit Wasserstoff und Ammoniak umsetzt.

Die reduktive Aminierung ist eine bekannte, in der Technik vielfach ausgeübte Reaktion zur Herstellung von Aminen. Einsatzstoffe für diesen Prozeß sind ausschließlich Aldehyde und Ketone. Die Umsetzung der Carbonylverbindungen mit Wasserstoff und Ammoniak erfolgt in Gegenwart von Hydrierkatalysatoren, insbesondere von Nickelträger-Katalysatoren. Die Umwandlung anderer Verbindungen nach dem Prinzip der reduktiven Aminierung, insbesondere die Umsetzung von Dioxanen, wurde bisher nicht beschrieben.

Obgleich 1,3-Dioxane formal als Acetale bzw. Ketale, d.h. als Umsetzungsprodukte von Aldehyden oder Ketonen mit 1,3-Diolen aufgefaßt werden können, lag es nicht nahe, diese Verbindungsklasse als Ausgangsstoffe für die Herstellung von Aminen einzusetzen. In diesem Zusammenhang ist zu berücksichtigen, daß in der synthetischen organischen Chemie Carbonylgruppen durch Reaktion mit Alkoholen, also durch Acetal- bzw. Ketalbildung geschützt werden, um sie vor weiteren Umsetzungen zu bewahren. Ferner handelt es sich bei den 1,3-Dioxanen um Sechsringverbindungen, die auf Grund der sterischen Gegebenheiten wie andere Sechsringe erhöhte Stabilität besitzen. Überraschenderweise gelingt es trotz dieser, einer Reaktion entgegenstehenden Voraussetzungen, Dioxane in die gewünschten 4-Oxa-Amine umzuwandeln. Die hohe Selektivität der Reaktion ist dabei besonders hervorzuheben, denn eine Spaltung des Moleküls in anderer Weise als unter Bildung einer 4-Oxa-Verbindung, z.B. unter Entstehung kleinerer Bruchstücke, ist nicht auszuschließen.

Bevorzugte 1,3-Dioxane nach der allgemeinen Formel (II) sind Verbindungen, in denen R¹, R², R⁴ und R⁵ gleich oder verschieden sind und Wasserstoff, geradkettige oder verzweigte Alkylreste mit 1 bis 12 und insbesondere 1 bis 6 Kohlenstoffatomen, Alkenyl- oder Alkinylreste mit 2 bis 12 und insbesondere 2 bis 6 Kohlenstoffatomen bedeuten. Bevorzugt sind ferner Verbindungen der allgemeinen Formel (II), in denen R¹, R², R⁴ und R⁵ für Cycloalkyl- oder Cycloalkenylreste mit 5 oder 6 Kohlenstoffatomen, für Aryl-, Alkylaryl-, Aralkyl-, Aralkenyl- oder Alkenylarylreste mit 6 bis 12 Kohlenstoffatomen und für heterocyclische Reste, die ein oder mehrere Stickstoff- und/oder Sauerstoff- und/oder Schwefelatome enthalten, stehen. Weiterhin werden 1,3-Dioxane der allgemeinen Formel (II) bevorzugt, in der R³ ein geradkettiger oder verzweigter Alkylrest mit 1 bis 12, insbesondere 1 bis 8, vorzugsweise 1 bis 4 Kohlenstoffatomen ist. Besonders bevorzugt steht R³ für Wasserstoff.

Beispiele für Alkyl-, Alkenyl- oder Alkinylreste sind der Methyl-, Ethyl-, n-Propyl-, i-Propyl-, Propenyl-, i-Propenyl-, n-Butyl-, i-Butyl, n-Butenyl, i-Butenyl, n-Butinyl-, Pentyl-, Pentenyl-, Pentinyl-, Hexyl-, Hexenyl-, Heptyl-, Heptenyl-, Octyl-, Octenyl-, Nonyl-, Nonenyl-, Decyl-, Decenyl-, Dodecyl- oder Dodecenylrest.

Cycloalkylreste werden beispielhaft durch den Cyclopentyl-, Cyclohexyl-, Cycloheptyl-, Cyclopentenyl- oder Cyclohexenylrest vertreten.

Als aromatische Reste kommen z.B. der Phenyl-, Benzyl-, 2-Phenylethyl-, 2-Phenylpropyl-, 3-Phenylpropyl-, 2-Phenylbutyl-, 3-Phenylbutyl-, 4-Phenylbutylrest in Betracht.

Beispiele für heterocyclische Reste sind der Furan-, Dihydrofuran-, Tetrahydrofuran-, Thiophen-, Dihydrothiophen-, Pyridin- und Thiopyranrest.

Die Alkyl-, Cycloalkyl-, aromatischen und heterocyclischen Reste können substituiert sein, insbesondere durch Reste, die unter den Reaktionsbedingungen inert sind wie Halogen, Alkoxy-, Carboxy- oder Carboxylatgruppen. Es ist aber in Einzelfällen auch nicht ausgeschlossen, daß bewußt Substituenten ausgewählt werden, die zu bestimmten Endprodukten reagieren.

Die als Ausgangsmaterial in das erfindungsgemäße Verfahren eingesetzten 1,3-Dioxane sind auf verschiedenen Wegen zugänglich. Ein bewährtes Verfahren ist die säurekatalysierte Addition von 1,3-Diolen an Aldehyde oder Ketone oder die Umacetalisierung von Acetalen bzw. Ketalen, insbesondere solchen, die sich von niedrig siedenden Alkoholen ableiten, mit 1,3-Diolen in Gegenwart von Säuren.

Geeignete Diolkomponenten sind z.B. Propandiol-1,3, 2-Methylpropandiol-1,3, 2-Ethylpropandiol-1,3, 2-Phenylpropandiol-1,3, 2,2-Dimethylpropandiol-1,3 (Neopentylglycol), 2,2-Diethylpropandiol-1,3, 2-Methyl-2-ethylpropandiol-1,3, 2-Methyl-2-propylpropandiol-1,3, 2-Methyl-2-butylpropandiol-1,3, 2-Methyl-2-phenylpropandiol-1,3, 2-Ethyl-2-butylpropandiol-1,3, 1,1-Dimethylolcyclohexan, 1,1-Dimethylolcyclopentan, 3,3-Dimethyloltetrahydrofuran, 3,3-Dimethyloltetrahydropyran und 2,2,4-Trimethylpentandiol-1,3.

Als Carbonylverbindungen werden mit den 1,3-Diolen z.B. aliphatische, aromatische oder heterocyclische Aldehyde und Ketone oder deren Acetale bzw. Ketale umgesetzt. Die Aldehyde können ebenso wie die Ketone, gesättigt oder ungesättigt sein.

Beispiele für geeignete aliphatische Aldehyde sind Formaldehyd, Acetaldehyd, Propionaldehyd, Butyraldehyd, Isobutyraldehyd, n-Pentanal, 2-Methylbutanal, 3-Methylbutanal, n-Hexanal, 2-Methylpentanal, 3,3-Dimethylbutanal, 2-Ethylhexanal, 2-Methyldecanal, ferner Dialdehyde wie Glyoxal, Methylglyoxal, Malondialdehyd, Succindialdehyd und Glutardialdehyd, sowie substituierte Aldehyde wie 3-Hydroxy-2,2-dimethylpropanal (Hydroxypivalaldehyd), Methoxypivalinaldehyd, Butoxypivalinaldehyd, 4-Acetoxybutyraldehyd und 5-Formylvalerianaldehyd.

Auch ungesättigte aliphatische Aldehyde können als Reaktionskomponente für die 1,3-Diole verwendet werden, z.B. Acrolein, α-Methylacrolein, α-Ethylacrolein und höhere α-Alkyl-, Isoalkyl- und Alkenylacroleine wie But-2-enal, But-3-enal, 2-Methylbut-2-enal, 2-Methylpent-2-enal, 2-Ethylhex-2-enal, 2,2-Dimethylpent-4-enal, 2-Methyl-4-acetoxy-but-2-enal, 2-Methoxymethylacrolein, 2-(3-Methoxycarbonylpropyl)-acrolein, 2-Methyl-4-chlor-but-2-enal.

Als aromatische Aldehyde seien beispielhaft genannt Benzaldehyd, p-Methoxybenzaldehyd, Phenylacetaldehyd, 2-Phenylpropanal, 3-Phenylpropanal, 2-Hydroxybenzaldehyd, 3-Hydroxy-4-methoxybenzaldehyd, Zimtaldehyd und Benzylacrolein.

Beispiele für heterocyclische Aldehyde sind Tetrahydrofuryl-2-aldehyd, Tetrahydrofuryl-3-aldehyd, Tetrahydrothienyl-2-aldehyd, Tetrahydrothienyl-3-aldehyd, 5,6-Dihydropyranyl-6-aldehyd, 2,5-Dimethyl-5,6-dihydropyranyl-6-aldehyd, Furyl-2-aldehyd, Furyl-3-aldehyd, Thienyl-3-aldehyd und 2-, 3- oder 4-Pyridylaldehyd.

Zur Herstellung der 1,3-Dioxane geeignete Ketone sind z.B. Aceton, Methylethylketon, Diethylketon, Methylisopropylketon, Diisopropylketon, Diisobutylketon, Methylisobutylketon, Methoxyaceton, Methylphenylketon, Methylisopropenylketon, Methylisobutenylketon, Cyclopentanon, Cyclohexanon, Dimethylcyclopentanon, Dimethylcyclohexanone, Cyclohexenon, 3,5,5-Trimethylcyclohexenon-2, Methyl-, Ethyl- und Vinylphenylketon, Methylfurylketon, Acetylaceton und Acetessigester.

Die vorstehend aufgeführten Diole, Aldehyde und Ketone als Bausteine für die erfindungsgemäß eingesetzten 1,3-Dioxane sollen lediglich die Anwendungsbreite des neuen Verfahrens skizzieren, es jedoch nicht auf diese beispielhaft genannten Verbindungen beschränken.

Andere Reaktionen, die zu 1,3-Dioxanen führen, sind die Umsetzung von Grignard-Reagenzien mit Orthoestern sowie die Reaktion von Alkoholaten von Diolen entweder mit geminalen Halogeniden oder mit α-Haloethern. Schließlich sind 1,3-Dioxane auch durch Prinsreaktion, d.h. die Addition eines Olefins an Formaldehyd in Gegenwart einer Säure, zugänglich.

Die reduktive Aminierung der 1,3-Dioxane nach dem erfindungsgemäßen Verfahren erfolgt bei 40 bis 500°C. Bevorzugt werden Temperaturen von 100 bis 450°C und insbesondere von 150 bis 350°C. Der Reaktionsdruck kann über einen weiten Bereich eingestellt werden und liegt bei 0,1 bis 35 MPa, wobei Drücke von 3 bis 15 MPa bevorzugt werden.

Das Molverhältnis von Ammoniak zu den 1,3-Dioxanen beträgt 0,2 : 1 bis 100 : 1. Verhältnisse von 0,5 : 1 bis 50 : 1 werden bevorzugt. Wasserstoff und 1,3-Dioxane werden in Molverhältnissen von 0,2 : 1 bis 250 : 1 und insbesondere in Verhältnissen von 1 : 1 bis 100 : 1 eingesetzt.

Die Umsetzung der Reaktanten erfolgt in Gegenwart von Hydrierkatalysatoren. Als hydrieraktive Komponente enthalten sie ein oder mehrere Metalle der Gruppen VIB, VIIIB und IB des Periodensystems der Elemente (entsprechend der von Chemical Abstracts verwendeten Gruppenbezeichnung, die auch weiterhin benutzt wird). Bevorzugt als hydrieraktive Komponenten werden Molybdän, Wolfram, Ruthenium, Kobalt, Rhodium, Iridium, Nickel, Palladium, Platin und Kupfer. Wenngleich diese Metalle auch in reiner Form, z.B. als Raney-Kobalt oder Raney-Nickel als Katalystoren Anwendung finden können, setzt man sie bevorzugt in Form von Trägerkatalysatoren ein.

Als Träger für die hydrieraktiven Metalle kommen acide Verbindungen oder Gemische acider Verbindung in Betracht. Besonders geeignet aus der Vielzahl dieser Substanzen sind Zeolithe. Unter dieser Bezeichnung versteht man kristalline, hydratisierte Alumosilikate mit Gerüststruktur, die Alkali- und/oder Erdalkalikationen enthalten. Sie kommen natürlich vor und werden auch synthetisch hergestellt. Zeolithe zeichnen sich strukturell durch ein regelmäßiges System aus interkristallinen Hohlräumen aus, das über Porenöffnungen für Moleküle gleicher Größenordnung zugänglich ist. Das Gerüst der Zeolithe besteht aus Tetraedern, wobei um das Zentralatom, das entweder ein Si⁴⁺- oder Al³⁺-Kation sein kann, vier Sauerstoffatome angeordnet sind. Jedes in die Struktur eingebaute Aluminiumatom führt zu einer negativen Ladung des Gerüsts, die durch Kationen, wie Alkali- doer Erdalkaliionen, ausgeglichen wird. Ein Austausch der Kationen ist möglich: Zeolithe sind anorganische Ionenaustauscher. So können z.B. Alkaliionen durch Wasserstoffionen ersetzt werden. Auf diese Weise läßt sich die katalytische Aktivität der Zeolithe variieren, die an das Vorliegen acider Zentren in der interkristallinen Oberfläche gebunden ist. Die Räume zwischen den Tetraedern sind von Wassermolekülen besetzt, eine Dehydratation durch Trocknen oder Calcinieren ist möglich.

Im Kristallgitter synthetisch gewonnener Zeolithe kann Aluminium durch andere Elemente, wie Bor, Gallium, Eisen, Chrom, Vanadium, Arsen, Antimon, isomorph ersetzt werden. Silicium läßt sich durch vierwertige Elemente, wie Germanium, Titan, Zirkon und Hafnium isomorph substituieren.

Art und Umfang des Austausches von Aluminium und/oder Silicium erlauben es, die katalytischen Eigenschaften der Zeolithe gezielt zu beeinflussen und individuellen Erfordernissen anzupassen.

Entsprechend ihrer Struktur unterteilt man die Zeolithe in verschiedene Gruppen. In den Zeolithen der Mordenit-Gruppe bilden die Grundeinheiten der Struktur, die SiO₄- und AlO₄-Tetraeder, Ketten. Die Zeolithe der Chabasit-Gruppe sind aus Tetraederschichten aufgebaut. Bei den Zeolithen der Faujasit-Gruppe sind die Tetraeder zu Polyedern angeordnet, z.B. in Form eines Kubooctaeders. Je nach Verknüpfung der Kubooctaeder entstehen unterschiedlich große Hohlräume und Poren, dementsprechend unterscheidet man z.B. Zeolithe vom Typ A, L, X oder Y.

Als Trägerkomponente für die im erfindungsgemäßen Verfahren eingesetzten Katalysatoren eignen sich Zeolithe aus der Faujasit-Gruppe, z.B. der Zeolith Y, Zeolithe aus der Mordenit-Gruppe oder engporige Zeolithe, z.B. vom Erionit- bzw. Chabasit-Typ. Bevorzugt werden Zeolithe vom Pentasil-Typ eingesetzt, die als Grundbaustein einen aus SiO₄-Tetraedern aufgebauten Fünfring besitzen und ein hohes SiO₂/Al₂O₃-Verhältnis aufweisen. Ihre Porengrößen liegen zwischen denen der Zeolithe vom Typ A (Porenöffnungen 4,1 Å) und denen vom Typ X oder Y (Porenöffnun-gen 7,4 Å). Die Pentasil-Zeolithe können chemisch unterschiedlich zusammengesetzt sein. Dementsprechend unterscheidet man Alumo-, Boro-, Eisen-, Gallium-, Chrom-, Arsen-, Antimon- und Bismutsilicatzeolithe oder deren Gemische, sowie Alumo-, Boro-, Gallium- und Eisengermanatzeolithe oder deren Gemische.

Vorzugsweise setzt man als Träger für die im erfindungsgemäßen Verfahren benutzten Katalysatoren Alumo-, Boro- und Eisensilikat-Zeolithe des Pentasil-Typs ein. Der Alumosilikat-Zeolith wird z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in einer Lösung von 1,6-Hexandiamin, 1,3-Propandiamin oder Triethylentetramin, gegebenenfalls unter Zusatz von Alkalimetall- oder Erdalkalimetallhydroxid bei 100 bis 220°C unter autogenem Druck hergestellt. Ein derartiges Verfahren ist in EP 0 007 081 und EP 0 007 098 beschrieben. Hierzu gehören auch die isotaktischen Zeolithe nach EP 0 034 727 und EP 0 046 504. Je nach Wahl der Einsatzstoffmengen beträgt in den synthetisierten Alumosilikat-Zeolithen das SiO₂/Al₂O₃-Verhältnis 10 bis 40.000 zu 1 (in mol). Nach einer anderen Arbeitsweise erhält man Alumosilikat-Zeolithe durch Umsetzung von Aluminium- und Siliciumkomponente in einem Ether, wie Diethylenglykoldimethylether, in einem Alkohol, wie Methanol oder 1,4-Butandiol oder in Wasser.

Borosilikat-Zeolithe kann man aus einer Borverbindung, z.B. H₃BO₃, und einer Siliciumverbindung, vorzugsweise hochdispersem Siliciumdioxid, in der wäßrigen Lösung eines Amins, insbesondere 1,6-Hexandiamin, 1,3-Propandiamin oder Triethylentetramin, gegebenenfalls unter Zusatz von Alkali- oder Erdalkalihydroxid, bei 90 bis 200°C unter autogenem Druck synthetisieren. Statt in wäßriger Aminlösung kann man in einem Ether, z.B. in Diethylenglykoldimethylether oder in einem Alkohol z.B. in 1,6-Hexandiol, als Lösungsmittel arbeiten (vgl. EP 0 007 081 und EP 0 007 098).

Zur Gewinnung der Eisensilikat-Zeolithe geht man z.B. von einer Eisenverbindung, vorzugsweise Fe₂(SO₄)₃, und einer Siliciumverbindung, vorzugsweise hochdispersem Siliciumdioxid, aus, die in der wäßrigen Lösung eines Amins, insbesondere 1,6-Hexandiamin, gegebenenfalls unter Zusatz von Alkali oder Erdalkalihydroxid, bei 100 bis 200°C unter autogenem Druck, umgesetzt werden (vgl. EP 0 007 081 und EP 0 007 098).

Die Alumo-, Boro- oder Eisensilikat-Zeolithe werden nach ihrer Herstellung und Isolierung bei 100 bis 160°C, vorzugsweise 110 bis 130°C, getrocknet und darauf bei 450° bis 550°, vorzugsweise 480 bis 520°C, calciniert. Anschließend werden sie unter Zusatz eines Bindemittels, z.B. zu Strängen oder Tabletten geformt. Als Bindemittel eignen sich die verschiedenen Aluminiumoxide, vorzugsweise Boehmit, amorphe Alumosilikate mit einem SiO₂/Al₂O₃-Gewichtsverhältnis von 0,3 : 1 bis 18 : 1, bevorzugt 3 : 1 bis 5 : 1, Siliciumdioxid, insbesondere hochdisperses SiO₂, Gemische aus hochdispersem SiO₂ und hochdispersem Al₂O₃, hochdisperses TiO₂ sowie Ton. Bindemittel und Zeolith werden im Gewichtsverhältnis 90 : 10 bis 40 : 60 verwendet. Nach der Formung werden die Formkörper erneut 10 bis 20 h bei 110 bis 130°C getrocknet und bei 400 bis 550°C zwischen 10 und 20 h calciniert.

Statt unmittelbar nach Herstellung, Isolierung und Trocknung zu calcinieren, können die Zeolithe auch getrocknet und geformt und im Anschluß daran calciniert werden. Schließlich ist es auch möglich, auf die Verwendung von Bindemitteln zu verzichten und die Formgebung unter Einsatz von Formgebungs- oder Peptisierungshilfsmitteln, wie Ethylcellulose, Stearinsäure, Kartoffelstärke, Ameisensäure, Oxalsäure, Essigsäure, Salpetersäure, Ammoniak, Amine, Silikoester oder Graphit oder deren Gemische, vorzunehmen.

Zeolithe, die auf Grund ihrer Herstellung Alkalimetall- oder Erdalkalimetallionen, jedoch keine oder nicht ausreichend viele H⁺-Ionen enthalten, müssen durch Ionenaustausch in die acide, katalytisch wirksame H-Form umgewandelt werden. Hierzu behandelt man sie mit Säuren oder führt Ammoniumionen ein und calciniert anschließend.

Durch abgestuften Ionenaustausch kann die für den speziellen Anwendungsfall erforderliche Acidität eingestellt werden.

Weiterhin können die Zeolithe durch Ionenaustausch oder durch Imprägnieren mit bestimmten Metallen modifiziert werden, um z.B. die Selektivität der Umsetzung zu verbessern oder die Katalysatorlebensdauer zu erhöhen. Bewährt hat sich die Dotierung der Zeolithe mit Übergangsmetallen der Gruppen VIB, VIIIB, IB, IIB, wie Chrom, Molybdän, Wolfram, Eisen, Nickel, Kupfer, Zink, mit Edelmetallen, wie Palladium und Platin und mit Metallen der Seltenen Erden, wie Lanthan, Cer und Praseodym.

Zur Dotierung durch Ionenaustausch behandelt man geformten oder nicht geformten Zeolith z.B. bei Temperaturen von 20 bis 100°C mit einer wäßrigen oder ammoniaklischen Lösung eines Salzes, beispielsweise eines Halogenids, Nitrats oder Acetats der vorbeschriebenen Metalle. Der Ionenaustausch kann mit Zeolithen in der Wasserstoff-, Ammonium- oder Alkalimetallform vorgenommen werden.

Beispielsweise legt man Stränge oder Pellets des Zeoliths in der H-Form in einer Kolonne vor und leitet eine ammoniakalische Pd(NO₃)₂-Lösung bei Temperaturen von 30 bis 80°C im Kreislauf während 15 bis 20 h über die Formkörper. Anschließend wird der Zeolith mit Wasser ausgewaschen, bei etwa 150°C getrocknet und bei etwa 550°C calciniert.

Nach einer anderen Variante des Ionenaustauschs suspendiert man pulverförmigen Zeolith in einer Metallsalzlösung, z.B. in einer ammoniakalischen Pd(NO₃)₂-Lösung und rührt bei 40 bis 100°C etwa 24 h. Nach Abfiltrieren, Trocknen bei etwa 150°C und Calcinieren bei etwa 500°C kann der modifizierte Zeolith mit oder ohne Bindemittel zu Strängen, zu Pellets oder zu Wirbelgut weiterverarbeitet werden.

Auch die Dotierung der Zeolithe durch Imprägnieren kann mit Metallsalzen, z.B. Chloriden, Nitraten oder Acetaten in wäßriger, ammoniakalischer oder alkoholischer Lösung erfolgen. Eine mögliche Ausführungsform besteht darin, daß man z.B. Wolframsäure, H₂WO₄, weitgehend in Wasser löst und mit dieser Lösung den geformten oder ungeformten Zeolith über einen bestimmten Zeitraum, z.B. 30 min, tränkt. Darauf entfernt man von der überstehenden Lösung durch Verdampfen das Wasser, trocknet den Zeolith bei etwa 150°C und calciniert bei etwa 550°C. Dieser Tränkvorgang kann mehrfach wiederholt werden, bis der gewünschte Metallgehalt eingestellt ist.

An die Dotierung der Zeolithe mit Metallen, unabhängig davon, ob sie durch Ionenaustausch oder durch Imprägnieren erfolgte, kann sich eine Nachbehandlung mit Wasserstoff anschließen.

Eine weitere Abwandlung der Zeolithe kann in einer Behandlung mit anorganischen oder organischen Säuren, wie Salzsäure, Flußsäure oder Phosphorsäure und/oder mit Wasserdampf bestehen.

Als Träger für Katalysatoren, die im erfindungsgemäßen Verfahren eingesetzt werden, haben sich ferner Phosphate bewährt. Besonders geeignet sind Aluminiumphosphate, Cerphosphate, Zirkonphosphate, Borphosphate, Eisenphosphate, Strontiumphosphate, Siliciumaluminiumphosphate, Siliciumeisenaluminiumphosphate oder deren Gemische.

Träger auf Basis von Aluminiumphosphaten, für die im neuen Verfahren angewandten Katalysatoren, erhält man vorteilhaft durch Synthese unter hydrothermalen Bedingungen. Zu diesen Aluminiumphosphaten gehören z.B. APO-5, APO-9, APO-11, APO-12, APO-14, APO-21, APO-25, APO-31 und APO-33. Diese Aluminiumphosphate besitzen Zeolith-Struktur (vgl. hierzu E.M. Flanigen et al., Structural Synthetic and Physicochemical Concepts in Aluminophosphate-based Molecular Sieves, Innovation in Zeolite Materials Science [Editors: P.J. Grobet et al.] Elsevier, 1988, p. 13 ff).

AlPO₄-5 (APO-5) erhält man beispielsweise durch Umsetzung einer mit Tetrapropylammoniumhydroxid versetzten homogenen Mischung von Orthophosphorsäure und Pseudoboehmit in Wasser in einem Autoklaven bei etwa 150°C und 20 bis 60 h Reaktionszeit unter autogenem Druck. Das abfiltrierte AlPO₄ wird bei 100 bis 160°C getrocknet und bei 450 bis 550°C calciniert.

AlPO₄-9 (APO-9) wird aus Orthophosphorsäure und Pseudoboehmit in einer wäßrigen 1,4-Diazadicyclo-(2,2,2)octan-Lösung bei etwa 200°C unter autogenem Druck und 200 bis 400 h Reaktionszeit synthetisiert.

Die Synthese von AlPO₄-21 (APO-21) erfolgt aus Orthophosphorsäure und Pseudoboehmit in wäßriger Pyrrolidin-Lösung bei 150 bis 200°C und 50 bis 200 h Reaktionszeit unter autogenem Druck.

Als Träger geeignete Aluminiumphosphate können auch durch Fällung erhalten werden. Man gewinnt sie beispielsweise, indem man zu einer Lösung von 92 g Diammoniumhydrogenphosphat in 700 ml Wasser innerhalb 2 h eine Lösung von 268 g Al(NO₃)₃xH₂O in 780 ml Wasser tropft. Durch gleichzeitige Zugabe von 25 %iger NH₃-Lösung hält man einen pH-Wert von 8 ein. Der entstandene Niederschlag wird 12 h nachgerührt, abgesaugt, ausgewaschen und 16 h bei 60°C getrocknet.

Beispiele für Siliciumaluminiumphosphate als Träger für die erfindungsgemäß eingesetzten Katalysatoren sind SAPO-5, SAPO-11, SAPO-31 und SAPO-34. Auch diese Siliciumaluminiumphosphate besitzen Zeolith-Struktur (vgl. hierzu R. Szostak et al., Catalysis Letters 2 (1989), p. 63 ff). Man erhält sie durch Umsetzung einer Mischung aus jeweils einer Silicium-, Aluminium- und Phosphorkomponente in wäßrigen, aminoorganischen Lösungen bei 100 bis 250°C unter autogenem Druck und 2 h bis 2 Wochen Reaktionszeit. SAPO-5 wird z.B. durch Mischen einer Suspension von SiO₂ in einer wäßrigen Tetrapropylammoniumhydroxid-Lösung mit einer Suspension aus Pseudoboehmit und Orthophosphorsäure in Wasser und anschließende Umsetzung bei 150 bis 200°C und 20 bis 200 h Reaktionszeit in einem Rührautoklaven unter autogenem Druck erhalten. Das abfiltrierte Pulver wird bei 110 bis 170°C getrocknet und bei 450 bis 550°C calciniert.

Andere geeignete Siliciumaluminiumphosphate sind z.B. auch ZYT-5, ZYT-6, ZYT-7, ZYT-9, ZYT-11 und ZYT-12.

Borphosphate, die als Träger für die im Verfahren eingesetzten Katalysatoren dienen können, gewinnt man z.B. durch Mischen und Kneten von konzentrierter Borsäure und Phosphorsäure, Trocknen des Gemischs und Calcinieren in Inertgas-, Luft- oder Dampfatmosphäre bei 250 bis 650°C, vorzugsweise 300 bis 500°C.

Auch die Träger auf Phosphatbasis können zur Erhöhung der Selektivität, des Umsatzes und der Lebensdauer modifiziert werden. Ein Weg hierzu ist die Dotierung der nicht geformten oder geformten Phosphate mit Metallsalzen durch Ionenaustausch oder Imprägnieren. Für die Dotierung setzt man Übergangsmetalle der Gruppen IVB bis VIIIB des Periodensystems, wie Titan, Zirkonium, Vanadium, Niobium, Chrom, Molybdän, Wolfram, Mangan, Rhenium, Eisen, Ruthenium, Osmium, Kobalt, Rhodium, Iridium, Nickel, Palladium, Platin, Übergangsmetalle der Gruppen IB und IIB des Periodensystems wie Kupfer, Silber, Zink, ferner Zinn, die Metalle der Seltenen Erden, wie Lanthan, Cer, Praseodym, Neodym, Erbium, Ytterbium und weiterhin Uran ein. Alkalimetalle, wie Lithium, Kalium, Caesium, Erdalkalimetalle, z.B. Magnesium, Calcium, Strontium, Metalle der Gruppen IIIA, IVA und VA des Periodensystems, wie Aluminium, Gallium, Germanium, Zinn, Blei, Bismut können als zusätzliche Promotoren im Trägermaterial bereits vorhanden sein oder eingebracht werden. Wie die Zeolithe können auch die Phosphate durch Behandlung mit anorganischen oder organischen Säuren abgewandelt werden.

Schließlich finden als Träger für Katalysatoren zur reduktiven Aminierung von 1,3-Dioxanen Metalloxide Anwendung, die acide oder amphotere Eigenschaften aufweisen. Geeignete Metalloxide sind z.B. die sauer wirkenden Oxide von Metallen der Gruppen IIIA und IVA und der Gruppen IVB bis VIB des Periodenystems, insbesondere Siliciumdioxid in Form von Kieselgel und Kieselgur, ferner Titanoxid, Zirkondioxid, die Phosphoroxide, Vandiumpentoxid, Nioboxide, Bortrioxid, Aluminiumoxid, Molybdänoxide, Wolframoxide und weiterhin Eisenoxide, für sich allein oder in Mischung aus zwei oder mehr dieser Verbindungen.

Es hat sich als vorteilhaft erwiesen, die genannten Oxide mit anorganischen oder organischen Säuren zu behandeln. Als anorganische Säuren kommen z.B. HF, HCl, HBr, HJ, H₂SO₄, H₂SO₃, HNO₃, H₃BO₃, die Phosphorsäuren und deren Mischungen in Betracht. Organische Säuren, die sich zur Behandlung der Oxide eignen, sind z.B. Ameisensäure, Essigsäure, Propionsäure und Oxalsäure allein oder in Mischung. Auch Gemische aus anorganischen und organischen Säuren können verwendet werden. Die Einwirkung der Säuren erfolgt auf das geformte oder nicht geformte Material.

SiO₂ (Silica) in Pulverform wird z.B. 1 h bei 80°C mit 1 n Säure behandelt. Danach wäscht man mit Wasser, trocknet 16 h bei 110°C und calciniert 20 h bei 500°C. Nach einer anderen Arbeitsweise behandelt man SiO₂ vor oder nach dem Formen 1 bis 3 h bei 60 bis 80°C mit 3 bis 25 Gew.-%igen, insbesondere 12 bis 20 Gew.-%igen wäßrigen Salzsäure, wäscht das SiO₂ anschließend mit Wasser, trocknet und calciniert bei 400 bis 500°C.

Nach einer besonders zweckmäßigen Ausführungsform behandelt man SiO₂ vor der Verformung durch Erhitzen mit 0,001 n bis 2 n, vorzugsweise 0,05 n bis 0,5 n Flußsäure z.B. unter Rückfluß im allgemeinen über einen Zeitraum von 0,5 bis 5 h, vorzugsweise 1 bis 3 h. Das Trägermaterial wird isoliert, ausgewaschen, zweckmäßig bei Temperaturen von 100 bis 160°C getrocknet und bei 450 bis 600°C calciniert. Nach einer anderen bevorzugten Ausführungsform der Säurebehandlung läßt man auf SiO₂ nach der Verformung bei erhöhter Temperatur, z.B. 50 bis 90°C, vorzugsweise 60 bis 80°C während 0,5 bis 5 h, vorzugsweise 1 bis 3 h, 12 bis 20 Gew.-%ige Salzsäure einwirken. Anschließend wäscht man das Material aus, trocknet es bei 100 bis 160°C und calciniert bei 450 bis 600°C. Der Behandlung mit Flußsäure kann sich auch eine Behandlung mit Salzsäure anschließen.

Phosphorsäure bringt man auf das Metalloxid-Trägermaterial z.B. SiO₂, Al₂O₃ oder TiO₂ durch Tränken oder Versprühen auf. So erhält man einen phosphorsäurehaltigen Träger beispielsweise durch Behandlung von SiO₂ mit H₃PO₄- oder NaH₂PO₄-Lösung und anschließendes Trocknen bzw. Calcinieren. Phosphorsäure kann auch zusammen mit Kieselgel in einem Sprühturm versprüht werden. Diesem Vorgang schließt sich eine Trocknung und meist eine Calcinierung an. Schließlich kann man Phosphorsäure in einer Imprägniermühle auf Siliciumdioxid aufsprühen.

Die vorstehend beschriebenen Trägermaterialien vom Zeolith-, Phosphat- und Metalloxidtyp sind Basis für die im erfindungsgemäßen Prozeß eingesetzten Katalysatoren. Hierzu müssen die Träger mit dem oder den hydrieraktiven Komponenten beladen werden. Wie bereits gesagt wurde, handelt es sich bei den hydrieraktiven Komponenten um Metalle der Gruppen VIB, VIIIB, IB und IIB des Periodensystems der Elemente.

Die Vereinigung von Träger und hydrieraktivem Metall kann auf verschiedenen Wegen erfolgen. Sind die Träger zum Ionenaustausch befähigt, dann behandelt man sie mit Lösungen der hydrieraktiven Metalle und ersetzt die austauschfähigen Kationen in der Kristallstruktur durch die Ionen der katalytisch wirksamen Metalle. Zweckmäßig setzt man Metallverbindungen ein, deren Anionen thermisch unbeständig sind und durch Erhitzen entfernt werden können, wie Acetat, Nitrat, Carbonat, Oxalat. Der Umfang des Ionenaustausches wird durch die Ionenaustauschisotherme bestimmt. Die Beladung der Träger mit dem aktiven Metall kann auch mit der Dotierung, wie sie vorstehend beschrieben wurde, verbunden werden, indem man für den Ionenaustausch Lösungen vorsieht, die sowohl Ionen des Hydrierals auch des Dotiermetalls enthalten.

In der Praxis führt man den Ionenaustausch in der Weise durch, daß man pulverförmige Molekularsiebe 1 bis 48 h, vorzugsweise 6 bis 36 h bei 20 bis 80°C mit einer ammoniakalischen Metallsalzlösung rührt. Aus der Differenz der Metallionenkonzentration vor und nach dem Ionenaustausch ergibt sich die Metallkonzentration auf dem Träger. Das mit dem Metall beladene Pulver wird mit destilliertem Wasser gewaschen, bei 110 bis 160° getrocknet und bei 400 bis 650°C calciniert unter Einhaltung einer Aufheizgeschindigkeit von 0,1 bis 10°C min⁻¹.

Ein anderes Verfahren zum Aufbringen der Hydrierkomponente besteht in der Tränkung des Katalysatorträgers mit der Metallsalzlösung. In der Praxis rührt man z.B. den Katalysatorträger bei 20 bis 80°C 1 bis 48 h, vorzugsweise 6 bis 36 h mit einer ammoniakalischen Lösung des Metallsalzes. Das Lösungsmittel wird abdestilliert, der beladene Träger bei 110 bis 160°C getrocknet und bei 400 bis 650°C calciniert. Auch im Falle des Tränkverfahrens können Aufbringen der hydrieraktiven Komponente und Dotierung des Trägers in einem Schritt durchgeführt werden.

Eine weitere Variante zur Herstellung von Hydrierkatalysatoren, die für das neue Verfahren geeignet sind, ist die gemeinsame Fällung von Hydrier- und Trägerkomponente. Hierbei sind zwei Möglichkeiten zu unterscheiden. Entweder wird die hydrieraktive Komponente auf den vorgefertigten Träger gefällt oder man fällt hydrieraktive Komponente und Träger gemeinsam aus. In der Praxis legt man z.B. die Metallsalzlösung, in der das Trägermaterial suspendiert ist, vor und fällt mit einem basischen Reagenz das Metall als schwerlösliche Verbindung, beispielsweise als Hydroxid, Hydrogencarbonat, Carbonat oder basisches Carbonat, auf den Träger aus. Bei der gleichzeitigen Fällung von Hydrier- und Trägerkomponente wird eine gemeinsame Lösung der Ausgangsverbindungen simultan mit dem Fällungsmittel umgesetzt. Das Fällungsgut wird gegebenenfalls bei Raum- oder erhöhter Temperatur nachgerührt, filtriert, gewaschen, getrocknet und calciniert.

Je nach dem angewandten Verfahren wird das Trägermaterial mit unterschiedlichen Mengen der hydrieraktiven Komponente beladen. Bei der Gewinnung von Katalysatoren durch Ionenaustausch ist die maximale Metallkonzentration durch das Austauschvermögen des Trägermaterials begrenzt. -Üblicherweise enthalten derartige Katalysatoren 0,5 bis 15 Gew.-% der hydrieraktiven Komponente, bezogen auf den Katalysator.

Bei Tränkungsverfahren kann man den Beladungsgrad des Trägers durch Variation der Konzentration der Metallsalzlösung und durch ein- oder mehrfache Wiederholung des Tränkungsvorganges über weite Bereiche variieren. Metallkonzentrationen von 0,1 bis 30, vorzugsweise 0,5 bis 10 und insbesondere 1 bis 5 Gew.-%, bezogen auf den Katalysator, können eingestellt werden.

Die größte Flexibilität hinsichtlich der Einstellung des Metallgehaltes im Katalysator erzielt man durch Anwendung von Fällungsverfahren, gleichgültig, ob die Metallkomponente auf den vorgefertigten Träger aufgebracht wird oder Metallkomponente und Trägerkomponente gemeinsam ausgefällt werden. Der gewünschte Metallgehalt kann bei dieser Arbeitsweise durch Wahl der Mengenverhältnisse von Metall und Träger frei bestimmt werden. Üblicherweise enthalten Fällungskatalysatoren, in Abhängigkeit vom ausgewählten Metall, 0,1 bis 30, vorzugsweise 0,5 bis 10 und insbesondere 1 bis 5 Gew.-% hydrieraktives Metall, bezogen auf den Katalysator.

Pulverförmige Katalysatoren können nach Isolierung, Trocknung und Calcinierung mit einem Bindemittel zu Strängen oder Tabletten geformt werden. Als Bindemittel eignen sich die verschiedenen Aluminiumoxide, vorzugsweise Boehmit, amorphe Alumosilikate mit einem SiO₂/Al₂O₃-Gew.-Verhältnis von 25 : 75 bis 90 : 5, bevorzugt 75 : 25, Siliciumdioxid, bevorzugt hochdisperses SiO₂, Gemische aus hochdispersem SiO₂ und hochdispersem Al₂O₃, TiO₂, ZrO₂ sowie Ton. Nach der Formung werden die Extrudate oder Preßlinge nochmals getrocknet und gegebenenfalls anschließend calciniert.

Statt sie mit einem Bindemittel zu versehen, können die pulverförmigen Katalysatoren auch unmittelbar nach dem Trocknen zu Tabletten oder Strängen geformt und darauf calciniert werden. Es hat sich bewährt, dem Katalysatorpulver Hilfsmittel zum Strangpressen oder Peptisieren zuzusetzen, z.B. Methylcellulose, Ethylcellulose, Stearinsäure, Kartoffelstärke, Ameisensäure, Essigsäure, Oxalsäure, Alkalilauge, Ammoniak, Amine oder Graphit.

Strang- und Tablettengröße richten sich nach den individuellen Erfordernissen. Üblicherweise setzt man die Katalysatoren als Stränge von 2 bis 4 mm, als Tabletten mit 3 bis 5 mm Durchmesser, als Pellets mit einer Kornfraktion von 1,0 bis 1,6 mm oder pulverförmig, z.B. auch als Wirbelgut mit Korngrößen zwischen 50 und 400 µm ein.

Das erfindungsgemäße Verfahren kann diskontinuierlich oder kontinuierlich betrieben werden. Die diskontinuierliche Reaktionsführung erfolgt in Autoklaven oder Druckrohren. Die kontinuierliche Betriebsweise kann in Festbett- oder Fließbettreaktoren erfolgen. Als Festbettreaktoren gelangen z.B. Schlaufenreaktoren, Hordenreaktoren, Kreisgasreaktoren und vorzugsweise Rohrreaktoren zum Einsatz. Zweckmäßigerweise beträgt bei Rohrreaktoren das Verhältnis von Reaktordurchmesser zu Katalysatorpellets 2 : 1 bis 20 : 1 und insbesondere 4 : 1 bis 10 : 1.

In den folgenden Beispielen wird das erfindungsgemäße Verfahren erläutert, jedoch nicht auf die beschriebenen Ausführungsformen beschränkt.

### Katalysator A:

Der Katalysator enthält 5 Gew.-% Kupfer (bezogen auf den Katalysator im nicht reduzierten Zustand) als hydrieraktives Metall und Borzeolith des Pentasiltyps als Träger.

Die Herstellung des Borzeoliths erfolgt durch hydrothermale Synthese. Hierzu läßt man 640 g hochdisperses SiO₂ und 122 g H₃BO₃ in Gegenwart von 8000 g einer wäßrigen 1,6-Hexandiamin-Lösung (Hexandiamin : Wasser = 1 : 1 in Gew.-teilen) bei 170°C unter autogenem Druck in einem Rührautoklaven miteinander reagieren. Das kristalline Umsetzungsprodukt wird abfiltriert, ausgewaschen, 24 h bei 100°C getrocknet und 24 h bei 500°C calciniert. Der Zeolith hat die Zusammensetzung 94,2 Gew.-% SiO₂ und 2,3 Gew.-% B₂O₃. Das pulverförmige Material wird unter Zusatz eines Verstrangungshilfsmittels zu Strängen geformt, die 16 h bei 110°C getrocknet und 24 h bei 500°C calciniert werden.

Die Beladung des Trägermaterials mit dem hydrieraktiven Metall erfolgt durch Imprägnieren, wobei eine wäßrige Cu(NO₃)₂.3H₂O- oder Cu(CH₃COO)₂.H₂O-Lösung, deren Kupfergehalt dem gewünschten Kupfergehalt des Katalysators entspricht, bei 70 bis 80°C 24 h über den Träger gepumpt wird. Die Behandlungszeit kann durch Zugabe von Ammoniak (als 25 %ige wäßrige Lösung) zur Metallsalzlösung, bis zur Einstellung eines pH-Wertes von etwa 10,5, deutlich verkürzt werden. Die Metallbeladung ist beendet, wenn die intensiv blaue Farbe der ammoniaklischen Kupfersalzlösung verschwunden ist.

Der imprägnierte Träger wird mit destilliertem Wasser gewaschen, 12 h bei 160°C getrocknet und 5 h bei 550°C calciniert.

### Katalysator B:

Katalysator B ist ein Produkt der Fa. Südchemie, München, das unter der Bezeichnung G69 in Tablettenform in den Handel gebracht wird. Er enthält etwa 50 Gew.-% Ni und etwa 2,4 Gew.-% ZrO₂ auf Kieselgur.

### Katalysator C:

Katalysator C enthält als Träger einen Zeolith des Typs H-BEA der Fa. PQ Corporation (im Handel unter der Produktbezeichnung Valfor CP-806B) mit einem molaren SiO₂/Al₂O₃-Verhältnis von 25. Der Träger wird mit einer Kupfersalzlösung imprägniert. Der Kupfergehalt des Katalysators beträgt 5 Gew.-% seine Oberfläche 750 m²/g.

### Katalysator D:

Katalysator D enthält als Träger einen Zeolith des US-Y-Typs, ein Handelsprodukt der Fa. PQ Corporation (Produktbezeichnung: CBV 600) mit einem molaren SiO₂/Al₂O₃-Verhältnis von 5,2. Der Träger wird mit einer Nickelnitrat-Lösung imprägniert. Der Nickelgehalt des Katalysators beträgt 10 Gew.-%, seine Oberfläche 660 m²/g.

### Katalysator E:

Katalysator E enthält als Träger einen Zeolith des US-Y Typs, ein Handelsprodukt der Fa. PQ Corporation (Produktbezeichnung: CBV 600) mit einem molaren SiO₂/Al₂O₃-Verhältnis von 5,2. Der Träger wird mit einer Nickelnitrat-Lösung imprägniert. Der Nickelgehalt des Katalysators beträgt 15 Gew.-%, seine Oberfläche 600 m²/g.

### Beispiele 1 bis 16

Die Umsetzungen werden unter isothermen Bedingungen in einem Rohrwendelreaktor (Durchmesser: 8 mm; Länge: 1000 mm) bei Reaktionszeiten von 1 bis 8 h durchgeführt. In dem Rohrreaktor befindet sich über dem Katalysatorbett eine Vorverdampferstrecke, um die Einsatzstoffe auf Reaktionstemperatur zu erwärmen. Das Reaktionsgemisch wird in einer Kühlfalle bei -33°C kondensiert, auf Raumtemperatur erwärmt und gaschromatographisch untersucht. Die Versuchsbedingungen und die Versuchsergebnisse sind in der nachfolgenden Tabelle zusammengefaßt.

**Tabelle**

| Reduktiven Aminierung von 5,5-Dimethyl-2-phenyl-1,3-dioxan (gelöst in 1,3-Dioxan im Molverhältnis 1 : 4) | | | | | | | | |
|---|---|---|---|---|---|---|---|---|
| Beispiel | Katalysator | T [°C] | P [MPa] | Dioxan/NH₃/H₂ [mol/mol/mol] | Belastung [g g⁻¹h⁻¹] | Dioxanumsatz [%] | Selektität bezüglich Amin [%] | Versuchsdauer [h] |
| 1 | A | 350 | 0,1 | 1:6:45 | 1 | 81 | 25 | 1 |
| 2 | A | 350 | 0,1 | 1:6:45 | 1 | 42 | 7 | 6 |
| 3 | A | 300 | 0,1 | 1:6:45 | 1 | 19 | 18 | 6 |
| 4 | B | 300 | 0,1 | 1:4:30 | 1 | 41 | 27 | 6 |
| 5 | B | 300 | 0,1 | 1:13:100 | 0,3 | 81 | 9 | 6 |
| 6 | B | 300 | 0,1 | 1:4:1 | 1 | 31 | 23 | 8 |
| 7 | B | 300 | 0,1 | 1:4:3 | 1 | 42 | 19 | 8 |
| 8 | B | 300 | 0,1 | 1:2:30 | 1 | 39 | 31 | 8 |
| 9 | B | 300 | 0,1 | 1:8:30 | 1 | 36 | 23 | 8 |
| 10 | B | 325 | 0,1 | 1:4:30 | 1 | 57 | 27 | 8 |
| 11 | B | 275 | 0,1 | 1:4:30 | 1 | 33 | 15 | 8 |
| 12 | C | 300 | 0,1 | 1:4:30 | 1 | 91,8 | 10,5 | 4 |
| 13 | D | 300 | 0,1 | 1:4:30 | 1 | 91,9 | 17,8 | 4 |
| 14 | E | 300 | 0,1 | 1:4:30 | 1 | 88,1 | 13,5 | 7 |
| 15 | E | 300 | 0,1 | 1:4:6 | 1 | 76,7 | 15,9 | 7 |
| 16 | E | 325 | 0,1 | 1:4:6 | 1 | 90,8 | 31,2 | 7 |

## Patentansprüche

1. Verfahren zur Herstellung von 4-Oxa-Aminen der allgemeinen Formel 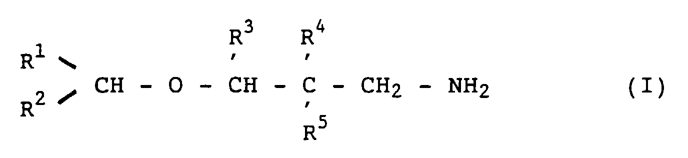 in der R¹, R², R⁴ und R⁵ gleich oder verschieden sind und Wasserstoff, geradkettige oder verzweigte Alkyl-, Alkenyl- oder Alkinylreste mit bis zu 18 Kohlenstoffatomen, Cycloalkyl- oder Cycloalkenylreste mit 5 bis 8 Kohlenstoffatomen, Aryl-, Alkylaryl-, Aralkyl-, Aralkenyl- oder Alkenylarylreste mit 6 bis 16 Kohlenstoffatomen oder heterocyclische Reste bedeuten, wobei jeweils die Reste R¹ und R² und/oder R⁴ und R⁵ zusammen mit dem Kohlenstoffatom, an das sie gebunden sind, ein Cycloalkan, Cycloalken oder einen Heterocyclus bilden können, R¹, R², R⁴ und R⁵ überdies unter den Reaktionsbedingungen inerte Substituenten tragen können und R³ für Wasserstoff oder einen geradkettigen oder verzweigten Alkylrest steht, dadurch gekennzeichnet, daß man 1,3-Dioxane der allgemeinen Formel (II) 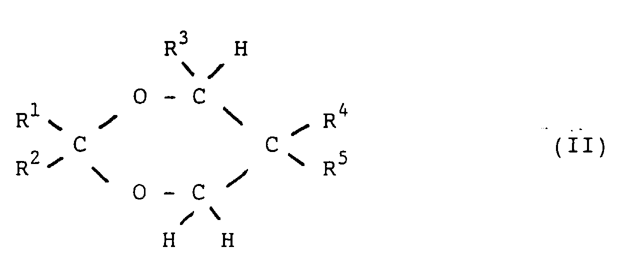 in der R¹, R², R³, R⁴ und R⁵ die oben angegebene Bedeutung haben bei Drücken von 0,1 bis 35 MPa und Temperaturen von 40 bis 500°C in Gegenwart eines Hydrierkatalysators mit Wasserstoff und Ammoniak umsetzt.

2. Verfahren nach Anspruch 1, dadurch gekennzeichnet, daß als 1,3-Dioxane Verbindungen der allgemeinen Formel (II) 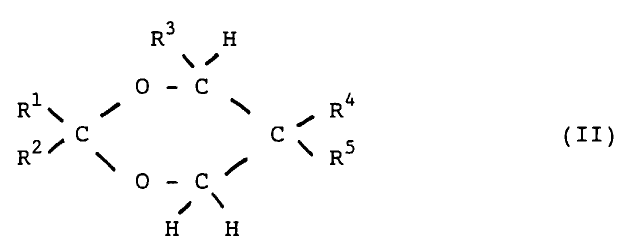 umgesetzt werden, in der R¹, R², R⁴ und R⁵ gleich oder verschieden sind und Wasserstoff, geradkettige oder verzweigte Alkylreste mit 1 bis 12 und insbesondere 1 bis 6 Kohlenstoffatomen, Alkenyl- oder Alkinylreste mit 2 bis 12 und insbesondere 2 bis 6 Kohlenstoffatomen, Cycloalkyl- oder Cycloalkenylreste mit 5 oder 6 Kohlenstoffatomen, Aryl-, Alkylaryl-, Aralkyl-, Aralkenyl- oder Alkenylarylreste mit 6 bis 12 Kohlenstoffatomen, heterocyclische Reste, die ein oder mehrere Stickstoff- und/oder Sauerstoff- und/oder Schwefelatome enthalten, bedeuten und R³ ein geradkettiger oder verzweigter Alkylrest mit 1 bis 12, insbesondere 1 bis 8, vorzugsweise 1 bis 4 Kohlenstoffatomen und besonders bevorzugt Wasserstoff ist.

3. Verfahren nach Anspruch 1 oder 2, dadurch-gekennzeichnet, daß als Hydrierkatalysatoren Trägerkatalysatoren eingesetzt werden.

4. Verfahren nach einem oder mehreren der Ansprüche 1 bis 3, dadurch gekennzeichnet, daß die Hydrierkatalysatoren als Träger Zeolithe enthalten.

5. Verfahren nach Anspruch 4, dadurch gekennzeichnet, daß der Träger ein Zeolith des Pentasil-Typs ist.

6. Verfahren nach Anspruch 4 oder 5, dadurch gekennzeichnet, daß der Zeolith ein Alumino-, Boro- oder Eisensilikatzeolith des Pentasil-Typs ist.

7. Verfahren nach einem oder mehreren der Ansprüche 4 bis 6, dadurch gekennzeichnet, daß der Zeolith mit Übergangsmetallen der Gruppen VIB, VIIIB, IB, IIB des Periodensystems dotiert ist.

8. Verfahren nach einem oder mehreren der Ansprüche 4 bis 7, dadurch gekennzeichnet, daß der Zeolith mit Edelmetallen dotiert ist.

9. Verfahren nach einem oder mehreren der Ansprüche 4 bis 6, dadurch gekennzeichnet, daß der Zeolith mit Metallen der Seltenen Erden dotiert ist.

10. Verfahren nach Anspruch 1 oder 2, dadurch gekennzeichnet, daß der Hydrierkatalysator als Träger Phosphate enthält.

11. Verfahren nach Anspruch 10, dadurch gekennzeichnet, daß der Hydrierkatalysator als Träger Aluminiumphosphat, Cerphosphat, Zirkonphosphat, Borphosphat, Eisenphosphat, Strontiumphosphat, Siliciumaluminiumphosphat, Siliciumeisenaluminiumphosphat, allein oder als Gemisch aus zwei oder mehr dieser Phosphate enthält.

12. Verfahren nach Anspruch 10 oder 11, dadurch gekennzeichnet, daß die Phosphate mit Übergangsmetallen der Gruppen IVB bis VIIIB, IB oder IIB oder mit Metallen der Seltenen Erden dotiert sind.

13. Verfahren nach Anspruch 1 oder 2, dadurch gekennzeichnet, daß der Hydrierkatalysator als Träger ein Metalloxid enthält.

14. Verfahren nach Anspruch 13, dadurch gekennzeichnet, daß der Träger ein Oxid von Metallen der Gruppen IIIA, IVA oder der Gruppen IVB bis VIB des Periodensystems oder ein Gemisch aus zwei oder mehr solcher Oxide ist.

15. Verfahren nach einem oder mehreren der Ansprüche 4 bis 14, dadurch gekennzeichnet, daß die Zeolithe, Phosphate und/oder Metalloxide mit anorganischen oder organischen Säuren behandelt sind.

16. Verfahren nach einem oder mehreren der Ansprüche 1 bis 15, dadurch gekennzeichnet, daß die Hydrierkatalysatoren als hydrieraktive Komponente ein Metall oder mehrerer Metalle der Gruppen VIB, VIIIB, IB oder IIB des Periodensystems enthalten.

17. Verfahren nach Anspruch 16, dadurch gekennzeichnet, daß die hydrieraktive Komponente Molybdän, Wolfram, Ruthenium, Eisen, Osmium Kobalt, Rhodium, Iridium, Nickel, Palladium, Platin, Kupfer, Silber oder Zink ist.

18. Verfahren nach einem oder mehreren der Ansprüche 1 bis 17, dadurch gekennzeichnet, daß die Umsetzung bei Drücken von 3 bis 15 MPa und bei Temperaturen von 100 bis 450°C, insbesondere 150 bis 350°C erfolgt.

19. Verfahren nach einem oder mehreren der Ansprüche 1 bis 18, dadurch gekennzeichnet, daß das Molverhältnis von Ammoniak zu dem 1,3-Dioxan 0,2 : 1 bis 100 : 1, vorzugsweise 0,5 : 1 bis 50 : 1, beträgt.

20. Verfahren nach einem oder mehreren der Ansprüche 1 bis 19, dadurch gekennzeichnet, daß das Molverhältnis von Wasserstoff zu 1,3-Dioxan 0,2 : 1 bis 250 : 1, insbesondere 1 : 1 bis 100 : 1, beträgt.
